# EUROPEAN PATENT SPECIFICATION

(11) **EP 4 322 963 B1**
(45) Date of publication and mention of the grant of the patent: **04.03.2026**
(21) Application number: 21745838.9
(22) Date of filing: 03.06.2021
(51) Int. Cl.: A61K 31/7048, A61P 31/14

(54) **TREATMENT OF AN INFECTION CAUSED BY SARS-COV-2 WITH NYSTATIN**
BEHANDLUNG EINER INFEKTION MIT SARS-COV-2 MIT NYSTATIN
MÉTHODE DE TRAITEMENT D'UNE INFECTION DE SARS-COV-2 PAR NYSTATINE

(30) Priority: 16.04.2021 HU 2100156; 16.04.2021 US 202163175874 P
(43) Date of publication of application: 21.02.2024
(73) Proprietor: Pannonpharma Gyógyszergyártó Kft., 7720 Pécsvárad (HU)
(72) Inventor: MILÁNKOVITS, Márton, 1112 Budapest (HU); PALLOS, József Péter, 1221 Budapest (HU); PÉNZES-HUVÖS, Ágota, 7720 Pécsvárad (HU); SEFFER, Dénes, 7720 Pécsvárad (HU); VARAJTI, Krisztina, 6300 Kalocsa (HU)
(74) Representative: Danubia Patent & Law Office LLC
(86) International application number: PCT/IB2021/054871
(87) International publication number: WO 2022/219386

(56) References cited:
- WO-A1-92/11841
- WO-A1-93/03737
- KADIOGLU ONAT ET AL: "Identification of novel compounds against three targets of SARS CoV-2 coronavirus by combined virtual screening and supervised machine learning", WORLD HEALTH ORGANIZATION, 21 March 2020 (2020-03-21), XP055814602, Retrieved from the Internet <URL:https://www.who.int/bulletin/online_first/20-255943.pdf> [retrieved on 20210616], DOI: 10.2471/BLT.20.255943
- AL-KHIKANI FALAHHASAN OBAYES: "Amphotericin B as antiviral drug: Possible efficacy against COVID-19", ANNALS OF THORACIC MEDICINE, vol. 15, no. 3, 1 January 2020 (2020-01-01), IN, pages 118, XP055872767, ISSN: 1817-1737, DOI: 10.4103/atm.ATM_147_20
- MANUS JEAN-MARIE: "La FDA approuve le remdesivir comme traitement de la Covid-19", REVUE FRANCOPHONE DES LABORATOIRES, ELSEVIER, AMSTERDAM, NL, vol. 2020, no. 527, 1 December 2020 (2020-12-01), pages 5, XP086395014, ISSN: 1773-035X, [retrieved on 20201208], DOI: 10.1016/S1773-035X(20)30320-8

## Description

### FIELD OF THE INVENTION

The invention relates to nystatin for use in the treatment of an infection caused by severe acute respiratory syndrome coronavirus 2 (SARS-CoV-2) in a human subject.

### TECHNICAL BACKGROUND OF THE INVENTION

### SARS-CoV-2

Viruses are intracellular parasites that modify cellular function for their own reproduction. Therefore, an unmissable step of the viral life cycle is the introduction of the viral genome into the cell. Lipid enveloped viruses deliver their genetic material into the cell in two steps: first, they bind to specific surface receptors on the membrane of the target cell and then, as a second step, the virus and the cell membrane are fused. (Más, V et al.)

SARS-CoV-2, like other coronaviruses, is an enveloped single stranded RNA virus consisting of a positive sense strand, which has four major structural proteins (S, E, N and M) and several accessory proteins (Jiang, S. et al.). Among them, protein S facilitates the attachment of the viral envelope to the host cell, while protein M determines the shape of the viral envelope. The virus uses ACE2 receptors of the host cell and transmembrane serine protease-2 (TMPRSS2) during the invasion of the host cell.

According to the literature, the virus can enter the body in the following ways:
- Via droplets, by inhalation of virus-containing particles, through nasal passages and eye-socket.
- Through the eye-socket, by rubbing the eye with virus-contaminated hand.
- Through the intestinal tract when eating with contaminated hands, touching the mouth, consuming food contaminated with droplets.

### Current therapeutic approaches for COVID

Currently, there are three therapeutic approaches to treat COVID-19 disease: systemic inactivation of the virus in the body; inhibition of viral replication within infected cells; and supportive therapy. The virus can be inactivated systemically in the body by vaccination (which leads to the development of a self repertoire of antibodies and cellular immunity) or by antibody therapy. The idea of antibody therapy is to extract and use antibodies produced during the disease as an immune response from the body of those who have already recovered. Plasma transfusion can assist in curing, rather than prevention, by delivering antibodies from outside the body, which can essentially work if the virus is present in the body.

Inhibition of virus replication within infected cells can be achieved by protease inhibitors or RNA polymerase inhibitors. One RNA polymerase inhibitor which is approved by the FDA for its use in the treatment of COVID-19 is remdesivir (see Manus, Jean.Marie: "La FDA approuve le remdesivir comme traitement de la COVID-19", Revue francophone des laboratoires, 2020, no 527, page 5).

Supportive therapy includes the use of anti-inflammatory drugs, immunomodulators, etc.

However, there is still an urgent need for a therapy that helps to prevent or reduce the severe symptoms of COVID-19 disease and the "post-covid" effects in SARS-CoV-2-positive patients.

### Digestive system and SARS-CoV-2

The virus infects numerous organs of the host, including the gastrointestinal system. ACE2 receptors and TMPRSS2 are also found in high abundance on luminal cells of the intestines, epithelial cells of the colon and enterocytes of the small intestine (Dong M. et al.).

Secondary infection of the gastrointestinal system can occur after ingestion of nasal secretions, lung secretions, or from the bloodstream, respectively. Studies suggest that, of the organs of the gastrointestinal system, the intestinal tract may be the most favourable environment for the virus. This is supported by studies describing the prolonged detection of viral load in faecal samples (Ghimire, S. et al.; Prakash, S. et al.).

Live virus (Wang, W. et al.; Xiao F. et al.), as well as virus inactivated by, for example, gastric acid or digestive enzymes, can be detected in faeces. This is possible because, after infection of the lungs or nasal cavity, secretions containing virus particles may be detected after ingestion without infection of the gastrointestinal system itself.

Although the symptoms of COVID-19 disease originate primarily from the respiratory organs, gastrointestinal symptoms are also common. In some of the infected patients, gastrointestinal symptoms appear before respiratory symptoms, and in others, gastrointestinal symptoms are present without respiratory symptoms (Zhang et al.). In faecal samples, the virus can still be detected even after the samples from the nasal and pharyngeal mucosa are negative (i.e. the virus has disappeared), and, moreover, the virus in the faecal sample can still be infectious (Gupta, S. et al.), i.e. the reservoir function of the intestine and faeces cannot be excluded even after the patient is no longer infectious according to the tests based on the usual (nasal and pharyngeal) samples.

Han et al. demonstrated that, in patients with gastrointestinal (GI) symptoms, there was a longer time between the onset of symptoms and viral clearance than in patients without GI symptoms. Jin X, Lian et al. found that a higher proportion of patients with GI symptoms developed fever above 38.5 ° C and/or had shortness of breath, headache, and fatigue. The available data suggest that, especially in mild to moderate cases, GI symptoms can be important markers of the progression and prognosis of the disease, and these symptoms should be taken into account in the treatment or the prevention of the spread of the disease, respectively.

Gupta et al. and Zuo et al. have shown significant changes in the intestinal microbiota of COVID-19 patients.

More specifically, in an article published in June 2020, Gupta and colleagues briefly reviewed the literature relevant to the detection of SARS-CoV-2 virus in the faeces of COVID-19 patients. They found that the virus was detectable in faeces 1-33 days after a negative nasopharyngeal secretion test. In a small case-control metagenomic study involving 15 patients, published in September 2020, Zuo et al. concluded that changes in the microbiota were associated with the amount of SARS-CoV-2 in the faeces and the severity of COVID-19 disease, and suggested that appropriate alterations in the intestinal microbiota might reduce the severity of the disease. However, no specific treatment other than dietary modification has been suggested.

Abu-Farha, M et al. suggest as a possibility that entry of the virus into the cell could be inhibited by the use of membrane-binding agents, as lipids are important part of both the metabolism and the membrane of the virus. The use of beta-cyclodextrin has also been suggested. However, they conclude that more work is needed in this area. The use of polyene macrolide antibiotics has not been mentioned.

### Nystatin

The antifungal agent nystatin has been discovered in the 1950s. It acts on the fungal cell membrane by binding to ergosterol, a membrane constituent, and forming an ion channel. To a lesser extent, nystatin also binds to cholesterol in the cell membrane of mammals, which causes its side effect. It is systemically toxic.

It is currently available in solution/suspension (for oral cavity), ointment (for skin and vagina) and tablet (for intestinal tract) dosage forms for the topical treatment of fungal infections. These dosage forms can be used because there is virtually no absorption observed from them.

In addition to the antifungal activity of nystatin, it is also known to have immunomodulatory and antiviral effects. Information on the *in vitro* antiviral activity of nystatin is rarely mentioned in the literature. In their experiments with beta-cyclodextrin and nystatin, Guyader et al. found that these compounds inhibit viral entry into the cell by reducing cholesterol levels in the viral membrane, and they suggested as early as 2002 that these compounds might be useful against HIV-1 and that, in addition to their *in vitro* studies, they might be worth testing in animal models. The compounds were hypothesized to inhibit caveolae-mediated endocytosis through their binding to cholesterol. However, the authors warn of the serious risk of systemic administration due to the toxicity of nystatin and consider only topical administration to be possible (Mireille Guyader et al.)

Nystatin has been used in clinical trials for antiviral indications only against HIV (https://clinicaltri-als.gov/ct2/show/record/NCT00002313), but it is not known that any drug has been developed for this indication.

According to a 2008 study, nystatin was not proved to be effective against SARS-CoV *in vivo* (Wang, H. et al.).

Kadioglu et al. (Identification of novel compounds against three targets of SARS CoV-2 coronavirus by combined virtual screening and supervised machine learning. World Health Organization, 21 March 2020 (2020-03-21)) describe nystatin - among several other compounds - as a possible candidate for treating Covid-19.

By treating SARS-CoV-2 virus-positive patients with orally administered nystatin, the inventors unexpectedly found that such treatment with a polyene macrolide antibiotic can alleviate the symptoms of COVID-19 disease and accelerate the recovery of patients.

### BRIEF DESCRIPTION OF THE INVENTION

The invention is defined by the claims.

Nystatin for use in the the treatment of an infection caused by severe acute respiratory syndrome coronavirus 2 (SARS-CoV-2) in a human subject is provided.

Preferably the treatment is carried out by oral and/or intranasal administration, preferably such that nystatin comes in contact with the entire digestive tract and/or respiratory tract, including the oral cavity, pharynx, oesophagus, nasal cavity and lungs.

Preferably the treatment is carried out by using rectal suppository.

Preferably nystatin is formulated with a cosolvent, preferably in combination with pyridoxal phosphate, polysiloxane, bile acid, modified bile acid, fulvic acid, humic acid and/or intralipid.

Preferably the subject is SARS-CoV-2 positive, preferably before the onset of symptoms or in the early stages of the disease, preferably wherein a replicable virus is detected in the digestive system of the subject.

Preferably nystatin is formulated for oral and/or intranasal administration, preferably in oral pharmaceutical form selected from the group consisting of tablets, preferably film coated tablets, capsules, gels, suspensions, solutions, powders and inhalable dosage forms.

Preferably nystatin is administered for at least 5 days and/or up to 120 days, preferably for 90 days, preferably for 5-60 days, very preferably for 7-30 days or 10-21 days.

Preferably nystatin is administered in a dose of at least 100,000, up to 1,000,000 international units (IU), preferably at least 200,000, up to 800,000 IU, very preferably at least 400,000, up to 600,000 IU, in particular about 500,000 IU.

Preferably nystatin is administered to the human subject according to the following therapeutic regimen: administering a dose of 400,000, up to 600,000 IU, in particular about 500,000 IU, for 5-60 days, very preferably for 7-30 days, more preferably for 10-21 days.

Preferably the SARS-CoV-2 virus is detectable in the faecal sample of the subject at the beginning of the treatment.

Preferably replicable SARS-CoV-2 virus is detectable in the faecal sample of the subject.

### BRIEF DESCRIPTION OF THE DRAWINGS

**Figure 1** shows a photograph of the "covid tongue" in patient 4.

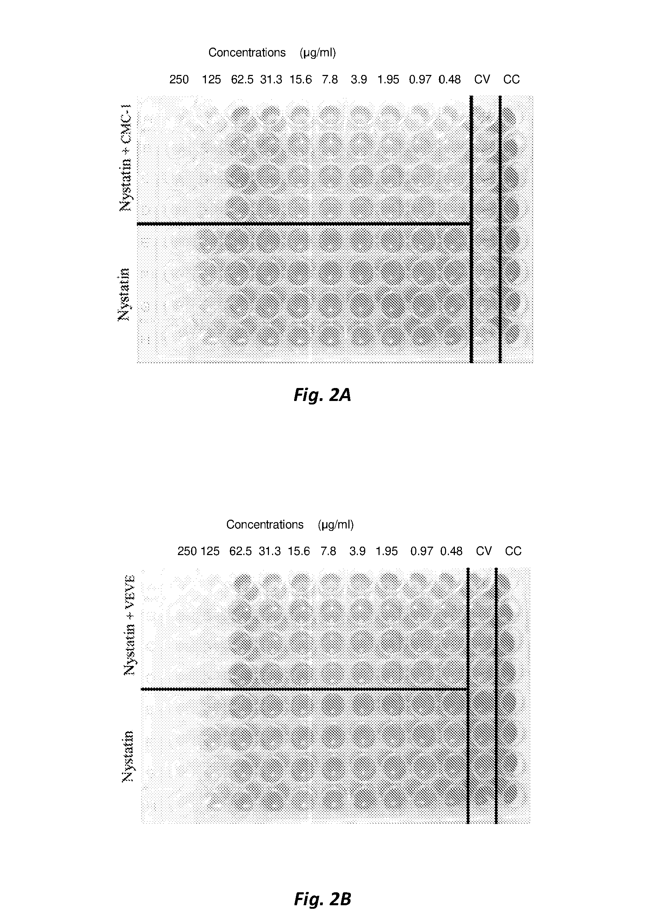

**Figure 2A** shows a photograph of a microtiter plate visualized using "neutral red" staining process. Tests were performed with VERO E6 cell line. To non-confluent cell cultures, serial dilution of Nystatin (E1-H10) and TCID₁₀₀ of the virus (early Wuhan strain CMC-1) pre-treated with different concentrations of nystatin (A1-D10) were added. In the column labeled CV, TCID₁₀₀ of untreated virus was added to the cultures, while no nystatin or virus was added to the cell cultures in the column labeled CC. As a result of the staining procedure, the colour of the supernatant is proportional to the viable cell number after incubation. The darker the colour is, the more living cells are present.
**Figure 2B** shows a photograph of a microtiter plate visualized using "neutral red" staining process. Tests were performed with VERO E6 cell line. To non-confluent cell cultures, serial dilution of Nystatin (E1-H10) and TCID₁₀₀ of the virus (British mutant strain VEVE) pre-treated with different concentrations of nystatin (A1-D10) were added. In the column labeled CV, TCID₁₀₀ of untreated virus was added to the cultures, while no nystatin or virus was added to the cell cultures in the column labeled CC. As a result of the staining procedure, the colour of the supernatant is proportional to the viable cell number after incubation. The darker the colour is, the more living cells are present.
**Figure 3A** is a graphical representation of the corrected absorbance values obtained from Table 1.
**Figure 3B** is a graphical representation of the corrected absorbance values obtained from Table 2.
**Figure 4A** is a microscopic image of a cell control (CC) tissue culture of Example 2 at the end of the incubation period, showing a confluent culture of cells with intact morphology.
**Figure 4B** shows a microscopic image of a virus control (VC) tissue culture of Example 2 at the end of the incubation period; no cells with intact morphology are visible.
**Figure 4C** shows a microscopic image of a tissue culture infected with CMC-1 virus of Example 2 at the end of the incubation period. Virus has been treated with 60 µg/ml nystatin. In the image, both cells with lysed and intact morphology can be seen.

### DETAILED DESCRIPTION OF THE INVENTION

In the present specification, the term "treatment" refers both to administer a medicine to a subject (mammal) to be treated ("treatment includes administration of....") and to changing a disease or condition in a subject so that the disease or condition is improved ("method of treatment", "use in a method for the treatment of an infection"). Treatment includes curative treatment, reduction of the severity, frequency, or number of symptoms and, optionally, prevention of the onset of symptoms as well. For example, in a subject infected with SARS-CoV-2, treatment may reduce the number of viruses in the subject's body (especially those in the digestive tract, particularly in the intestinal tract), relieve symptoms (e.g. diarrhea, vomiting) or prevent the onset of certain symptoms (e.g. digestive-system symptoms, "post-COVID" symptoms, "long covid" symptoms).

In the specification, "SARS-CoV-2 positive subject" means a person who is infected with SARS-CoV-2 and/or in whose body SARS-CoV-2 is detectable.

In the specification, the term "subject" is used for mammals.

It is generally known that the microbiota play a fundamental role in the development and functioning of the immune system and, additionally, influences immune responses elsewhere in the body, such as in the lungs (via the so-called gut-lung axis), i.e. the intestinal microbiota or changes in it can also influence respiratory diseases (Anand, S. et al.).

Intestinal microbiota play a role in the development and regulation of local and non-local inflammatory responses.

The intestinal microbiota can also influence the progress of viral infection itself. Geva-Zatorsky and colleagues, for example, have shown that Coprobacillus spp. increase ACE2 production in the intestinal tract of mice. Zuo et al. have shown that faecal samples from high SARS-CoV-2-infected individuals had higher levels of *Collinsella aerofaciens, Collinsella tanakaei, Streptococcus infantis, Morganella morganii,* while samples from low SARS-CoV-2-infected or uninfected individuals had higher levels of *Parabacteroides merdae, Bacteroides stercoris, Alistipes onderdonkii* and *Lachnospiraceae bacterium* 1_1_57FAA. It has also been shown that the virus was present (in infectious form) in the intestinal tract of infected individuals even when the patient did not show GI symptoms. The results of Kim and colleagues suggest that the 3' end of the SARS-CoV-2 genome (where coding regions of the S, E, M and N proteins are located) is of particular importance in the life cycle of the virus during active replication and transcription of SARS-CoV-2. Zuo and colleagues found higher sequencing coverage of the 3' end in faecal samples from infected individuals without GI symptoms.

It has been suggested that COVID-19-induced dysbiosis in the intestines may therefore cause long-lasting symptoms even after the virus has been cleared from the body. The gastrointestinal system, in particular the condition of the intestinal microbiota, may play a role in the course of the disease.
∘ For example, in case of involvement of the gastrointestinal system, the immune system is affected. This may have an adverse effect on the course of the disease.
∘ Once infected, the gastrointestinal system can act as a focal point for virus replication and spread in the body.

However, this has not been proven and the prior art does not provide any guidance as to what treatment of the intestinal tract could be beneficial for COVID-19 patients. Treatment with antibiotics seems to be particularly contraindicated, but it is used in daily therapy because of the bacterial superinfection of pneumonia.

In the patients we treated, we unexpectedly found that the treatment with nystatin, which could only affect the amount of coronavirus replicating in the digestive tract (including the oral cavity, pharynx and oesophagus), had a beneficial effect on the overall course of the disease, alleviating the course of the disease.

### Polyene macrolides

Polyene macrolides damage the envelope of viruses, thus reducing their ability to replicate (Guyader et al.). Examples of drugs belonging to the group of polyene macrolides include amphotericin B, natamycin, nystatin and pimaricin.

Amphotericin B can be nephrotoxic when administered intravenously, so using a tablet formulation is preferred. Natamycin is a natural antimicrobial organic compound produced by the bacterium *Streptomyces natalensis.* Natamycin is well tolerated by the skin and mucous membranes, even when they are inflamed. For blepharitis, conjunctivitis and keratitis, it is administered in the form of an eye ointment in adults. Treatment regimen in adults: 5% suspension eye drop; 1 drop in the conjunctival sac every 1-2 hours, then reduced to 1 drop 6-8 times daily after 3-4 days. Duration of treatment is 2-3 weeks. Natamycin is a fungicide. The virus causes conjunctivitis in ten percent of individuals infected with coronavirus. Natamycin is suitable for targeted topical treatment of this condition (Güemes-Villahoz, N. et al.).

### Nystatin

It is an antifungal compound belonging to the group of polyene macrolides. Nystatin has a favourable side effect profile, partly because it is poorly absorbed from the digestive tract. It has the following formula:

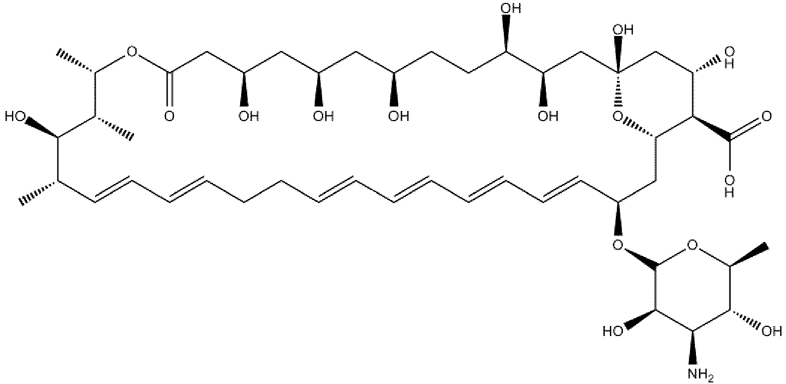

Polyene macrolides (amphotericin B and nystatin) have limited solubility. Increasing the solubility of polyene macrolides may improve their biological effect. The solubility can be increased by the use of pyridoxal phosphate, also known as vitamin B6, as it is assumed that a hydrogen bond is formed between the primary amine groups in the native form of the polyene macrolides and the 5'OH group of vitamin B6. Based on this principle, Day, TP and co-workers have successfully increased the solubility of polyene macrolides. To increase the bioavailability of nystatin, the use of other cosolvents known in the art may be beneficial, such as polysiloxane, bile acids and their derivatives, fulvic acid, humic acid or intralipid (Carmen Racles et al.; Pavlović N. et al.; Semis R. et al.). The increase in bioavailability can be only to the extent necessary to trigger the local effect without reaching systemic toxic concentrations. Although it is reported in the literature that polyene macrolides, such as nystatin, may be able to alter mammalian cell lipid metabolism and thus interfere with viral replication, it does not follow from this that they have a beneficial effect in the case of viral infection. Previous attempts to treat such diseases with nystatin have failed (see above under "Technical background").

The virus that escapes from the infected cell forms its own envelope from the cell membrane. In this way, the viral envelope is in fact the membrane of the infected cell, and thus contains cholesterol. Therefore, nystatin can also bind to the viral particle, so it can block the virus-host cell contact from the virus side.

Treatment with nystatin reduces the coronavirus burden, presumably by affecting the lipid composition of the viral envelope, thus mitigating the course of the infection and reducing the number of deaths.

In most cases, the virus replicates not only in the intestines but also in the respiratory tract, so systemic treatment may be beneficial in addition to nystatin.

The active agent nystatin may play several different roles in COVID-19 therapy.
1.) Its original indication is antifungal activity. It is used in weakened patients in intensive care units according to literature (Corchueloa et al.). However, it is not considered to be a part of the invention. Group of patients suffering from fungal infection of the gastrointestinal system, preferably the intestinal tract, is optionally excluded from the scope of the claimed invention. Furthermore, group of patients at risk of fungal infection of the gastrointestinal tract, preferably the intestinal tract, who have received preventive nystatin treatment, and who may optionally require such treatment, will also be optionally excluded.
2.) Inhibition of virus replication in the digestive tract.
3.) Effects of nystatin on cells, optionally on cells of the patient's digestive tract, optionally on the intestinal flora (microbiota), including immunomodulatory effects.

Our PCR-test positive patients treated with nystatin also had resolution of complaints arising away from parts of the digestive tract, despite the minimal absorption of nystatin from the intestines. The treatment reduces the viral load in the digestive tract, resulting in the disappearance of complaints such as fever, joint pain, weakness, lethargy, cough, etc. within a few days, much sooner than it would be expected without treatment. The beneficial effect of nystatin in the treatment of COVID-19 disease, in addition to its antiviral activity, is also complemented by its ability to inhibit the emergence of common secondary opportunistic infections (Corchuelo J. et al.).

### Diagnostics

### Detection of SARS-CoV-2 in faecal samples

SARS-CoV-2 viral load in faecal samples can be detected by conventional PCR assays (real-time reverse transcriptase), e.g. as described by Zuo et al, Zuo et al, Wang et al, Papoutsis et al, Coryell et al and Szymczak et al.

Detection of SARS-CoV-2 from faecal samples is not different in basic methodology from detection from other (mucous) samples, but the sample must be properly prepared. Sample preparation can be done, for example, as described in the above mentioned references, for example by suspension, dilution, homogenisation, clarification, centrifugation and/or filtration of the faecal sample in physiological saline, PBS or virus transfer medium. Viral RNA can be extracted using e.g. the QIAamp Viral RNA mini kit (Qiagen, Germantown, MD, USA or Hilden, Germany) or the Xpert Xpress SARS-CoV-2 or Hologic Panther Fusion assay. PCR can be performed using e.g. the 2019_nCoV RUO testing kit. For more information on the tests, see e.g. SARS-CoV-2 US Centers for Disease Control and Prevention (CDC) EUA protocol (appendix 1 p 3). The list of applicable *in vitro* diagnostic devices, their characteristics and instructions for use can be found on the relevant European Council website (https://covid-19-diagnostics.jrc.ec.europa.eu/devices).

### Detection of the presence of virulent SARS-CoV-2

The presence of virulent SARS-CoV-2 is indicated when the coverage of the 3' end of the viral genome (e.g. the S, E, N, M protein coding sequences or part of them) is higher than the coverage of the 5' end (active viral infection signature). The active viral signature can be detected e.g. as described by Zuo et al.

### Detection of SARS-CoV-2 infection

In addition to detection of viral RNA by PCR, infection can be detected by identification of specific antibodies (e.g. by ELISA-based methods), by immunochemical detection of antigen or by imaging techniques (e.g. lung CT). The list of applicable *in vitro* diagnostic devices, their characteristics and instructions for use can be found on the relevant European Council website (https://covid-19-diagnostics.jrc.ec.europa.eu/devices).

By using cheap and quick antigen test, the presence of infection, the viral load and the clearance of the virus from the body can be equally detected. Information on the use of antigen tests can be found on the US Centers for Disease Control and Prevention website (https://www.cdc.gov/coronavirus/2019-ncov/lab/resources/antigen-tests-guidelines.html; downloaded 13.04.2021) and on the corresponding European Council website (https://covid-19-diagnostics.jrc.ec.europa.eu/devices; downloaded 13.04.2021). For example, the Novel SARS-CoV-2 Coronavirus Spike Glycoprotein Detection Kit (S Protein-ACE2 receptor) from Apollo Biomedical LLC allows detection of antigen from faeces

The tests can even be used to monitor the effect of antiviral treatment, as long as the samples are analysed at appropriate intervals during the treatment period.

### Mechanism

The invention is not intended to be limited to any hypothetical mechanism of action, but it is believed that nystatin or polyene macrolides have the following mechanism of action.

Nystatin belongs to the group of polyene macrolides. The following relates mainly to nystatin, but also to other polyene macrolides in general.
- They form an ion channel, so they can destroy a variety of cells.
- They bind to sterols in the cell membrane to get into the membrane.
- They have different binding affinities for different sterols. Nystatin binds more strongly to ergosterol (Silva et al.), found in the fungal membrane, than to cholesterol, found in the membrane of mammalian cells, and therefore is used in antifungal therapy.
- When administered orally, nystatin is not absorbed and has no systemic effect from this pharmaceutical form and can therefore be used to treat fungal infections of the gastrointestinal system.
- According to a substantial part of the literature, nystatin exerts its antiviral effect not directly on the virus, but on the cell to be infected by the virus. That is, it modifies the cell surface, not the virus, through lipid rafts "floating" on the cell surface, and, linked to their cholesterol content, prevents the virus particle from entering the cell. This requires a high concentration of the active substance that would be very difficult to deliver systemically because of toxicity.
- In the literature, it has been suggested that polyene macrolides, such as nystatin, may be able to alter lipid metabolism of mammalian cell, thereby interfering with viral replication. However, this depends so much on the virus, host and active substance that, even if this were true in this case, it is certainly not clear to the person skilled in the art ("cholesterol depletion").
- The SARS-CoV-2 virus is a lipid enveloped virus. The virus that escapes from an infected cell forms its own envelope from the cell membrane. Thus, in effect, the viral envelope is the membrane of the infected cell, and thus contains cholesterol. Therefore, nystatin can also bind to the viral particle. According to a smaller part of the literature, it can thus block the viral-host cell contact from the viral side as well.

The virus can multiply in the body even when it is no longer present in the respiratory tract. This can cause a condition called post-acute covid. Persistent coronavirus infection in children may also be the cause of severe multisystem inflammatory syndrome in children (MIS-C). This is a serious disease that appears to be linked to COVID-19. Rarely, a similar syndrome occurs in adults: this new and severe syndrome has been named multisystem inflammatory syndrome in adults (MIS-A), which occurs in adults who have previously been patients with COVID-19. There is currently no known treatment for either MIS-C or MIS-A.

There, it is possible that the coronavirus may have disappeared from the respiratory tract, but still present in the gastrointestinal system, especially in the intestines. This in turn could potentially allow the coronavirus to re-infect other organs at a later stage, so that a patient with relieving symptoms could be getting worse again. Polyene macrolides, in particular nystatin, are thought to help prevent this and may also be effective in treating the aforementioned disease.

Polyene macrolides, in particular nystatin, may also indirectly contribute to the treatment of coronavirus related diseases by eliminating the virus from the gastrointestinal system and restoring healthy intestinal flora.

### In vitro demonstration of the anti-SARS-CoV-2 activity of nystatin

The *in vitro* demonstration of the anti-SARS-CoV-2 activity of nystatin was based on the method developed by Alessandro Manenti and co-workers to measure the viral neutralizing activity of human serum samples, with slight modifications. Tests were performed on VERO E6 cell line with two different SARS-CoV-2 strains, the early Wuhan (CMC-1) and the British mutant (VEVE).

The current standard use of nystatin for the treatment of fungal infections of the intestinal tract is 2-3 tablets of 500,000 IU (100 mg) 3 times daily, which provides a safe therapy. Nystatin, when administered as usual, is locally passed from the stomach to the intestine at concentrations in excess of 300 µg/ml and is considered stable during treatment.

In the study, the effect of nystatin was tested starting from a concentration of 250 µg/ml nystatin, using a half-dilution series in suspensions containing a constant TCID₁₀₀ virus dose (TCID₁₀₀ stands for 100% tissue culture infective dose, which is the amount of virus that can infect and kill all cells of the cell culture used). Virus was treated with nystatin in a separate plate (37 °C; 0.5 h). Subsequently, the nutrient solution on the non-confluent VERO E6 cell line was changed to nutrient solutions containing the nystatin-treated virus, and the plates were cultured for 3 days at 37 °C in a thermostat under atmosphere containing 5% CO₂.

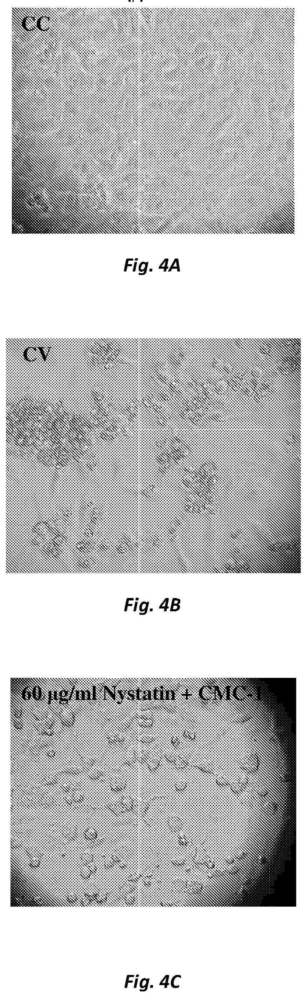

At the end of the incubation period, the tissue cultures were first examined under an inverted microscope and then stained according to the literature. Where the corrected absorbance was greater than 0.05, viable cells with intact morphology were observable by microscopy at the end of the study (see Figure 4C). Untreated TCID₁₀₀ virus used as a control caused complete cell death in tissue culture (Figure 4B).

According to the study performed, the IC₅₀ for nystatin is 60 µg/ml for the Wuhan strain and 125 µg/ml for the British mutant (detailed experimental data are presented in Example 2).

### Dosing (dose and duration)

The duration of treatment with nystatin tablets can range from a few days to three weeks, preferably 5 to 20 or 5 to 15 days, particularly preferably 10 to 14 days. The daily dose of nystatin is 3 x 500,000 to 1,000,000 IU per day for adults, or 2x or 4x 500,000 to 1,000,000 IU per day for adults. Dosing can be modified according to the patient's condition.

The dose of nystatin is 3x2 tablets for 10 days, but there have been cases that required 3 weeks of treatment. Dosage and treatment duration can be adjusted depending on the patient's condition.

If the patient's condition requires systemic treatment, particularly because of severe respiratory symptoms, systemic amphotericin B tablets should be used in addition to the treatment. The aim is to reduce the infectivity, the viral load and the severity of the condition, and to prevent or reduce the cytokine storm and the severe organ damage associated with it.

### Target group

The treatment can be used in all subjects infected with SARS-CoV-2 who are not allergic, intolerant or otherwise contraindicated to the formulation to be used. In particular, the target group for treatment includes those in whom SARS-CoV-2 infection has been confirmed by PCR, antigen test or antibody testing. Preferably, detection can also be made from a faecal sample. It is preferred if the presence of SARS-CoV-2 in a sample other than faeces (e.g. mucosal smear from nasal cavity or pharynx, or saliva) in the subject to be treated is no longer detectable, but is still detectable in the faecal sample. Preferably, infectious SARS-CoV-2 can be detected in faecal samples. The treatment can also be used in infected subjects who are asymptomatic or have symptoms other than gastrointestinal symptoms and as a preventive treatment. Preferably, the subject to be treated has gastrointestinal symptoms (e.g. vomiting, diarrhea).

### EXAMPLES

### Example 1

### Treatment of patients

Below are specific cases based on the records of one of the inventors, a physician practicing in Budapest. The identifying data of the physician and patients are known and clear, but are not included herein for privacy reasons.

### Patient 1

Male, date of birth: 08 Jan 1952, place of residence: Budapest, other illness: diabetes mellitus for 2-3 years.

In September 2020 he has fever, diarrhea and tested positive for Covid-19. He took 3x2 Nystatin tablets (500,000 IU film-coated tablets) per day for 10 days. Initially, he was weak, fatigable and had limb pain, stomach pain and headache. During the treatment, his complaints steadily decreased and disappeared after 10 days.

### Patient 2

Male, date of birth: 22 Dec 1981, place of residence: Budapest, other illness: diabetes mellitus for 2-3 years.

In the beginning of March 2021, he started to suffer from fever and loss of appetite. He coughed and tested positive for coronavirus by PCR. On 12 March, he started to take 3x2 Nystatin tablets (500,000 IU film-coated tablets) per day for 15 days. During the course of taking the tablets, he experienced a steady improvement in his condition and his appetite was restored. By the end of the treatment he was free of complaints.

### Patient 3

Female, date of birth: 25 Jul 1958, place of residence: Budapest

At the beginning of March 2021, she developed fever and loss of appetite. She coughed and tested positive for coronavirus by PCR. On 12 March she started taking 3x2 Nystatin tablets (500,000 IU film-coated tablets) per day for 15 days. Initially she was tired and had loss of appetite, then her appetite slowly came back, her coughing and choking subsided, her fatigue eased. By the end of the treatment she was free of complaints.

### Patient 4

Male, date of birth: 02 Jun 1953, place of residence: Gy 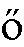 rzámoly. Weight: 110 kg, height: 188 cm, strong build.

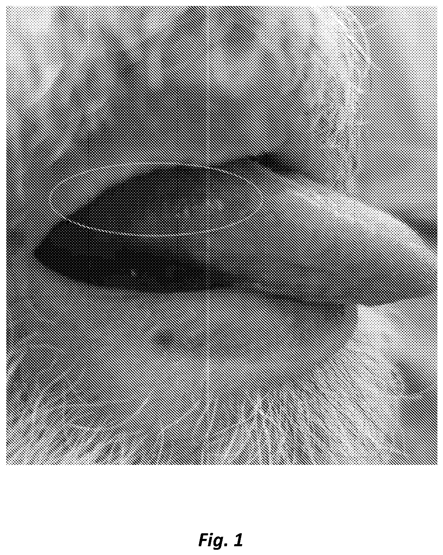

01 Apr 2021: onset of illness with severe headache, 02 Apr 2021: also severe headache, accompanied by diarrhea. He lost his sense of smell and taste and has a fever. 03 Apr 2021: covid test positive. 04 Apr 2021: febrile state with loss of appetite, stools few, dilute. 05 Apr 2021: febrile state with loss of appetite, stools few, dilute. 06 Apr 2021: febrile, loss of appetite, stools few, dilute. 07 Apr 2021: febrile, loss of appetite, stools few, dilute. For several days during this period he had a high fever of 39-39.8 °C, with dry coughing almost all day. He could eat almost nothing, he was drinking liquids, but became very weak over a few days. He was in bed almost all day, but he could not sleep much. "Covid-tongue" oral cavity phenomenon (Fig. 1) was developed. 08 Apr 2021: febrile, loss of appetite, stools few, dilute, Nystatin 2x2 tablets in the afternoon, evening. Further Nystatin therapy 3x2 tablets daily, one of which was sucked orally and dispersed, for oral disinfection. 09 Apr 2021: fever reduced to 37.5 °C (day 9), stools few, soft. 10 Apr 2021: subfebrility 36.9 °C (day 10), stools medium, soft, improved taste and appetite. 11 Apr 2021: Normal body temperature (day 11) 36.3 °C, stools normal, slightly soft, sense of taste, appetite returned, he was feeling well. 14 Apr 2021: "post-covid" asymptomatic state.

### Example 2

### In vitro demonstration of the effect of nystatin against SARS-CoV-2

The study was based on the method developed by Manenti and colleagues to measure the virus neutralising effect of human serum samples, with slight modifications. Studies were performed on VERO E6 cell line with two different SARS-CoV-2 strains: early Wuhan (CMC-1) and the British mutant (VEVE). The VERO E6 cell line used in the study was grown and maintained in DMEM (Dulbecco's Modified Eagle's Medium) supplemented with 2mM L-Glutamine, 100 units/mL penicillin-streptomycin mixture and 10% FBS, according to standard tissue culture procedures.

In this study, the antiviral effect of nystatin was tested starting from a concentration of 250 µg/ml nystatin, using a half-dilution series in suspensions containing constant TCID₁₀₀ virus quantity. Virus was treated with nystatin in a separate plate (37 °C; 0.5 h). Subsequently, the culture medium on the not yet confluent VERO E6 cells was exchanged for the medium containing the nystatin-treated virus, and the plates were incubated for 3 days at 37 °C under 5% CO₂.

At the end of the incubation period, the tissue cultures were examined by inverted microscopy (Figures 4A, 4B, 4C) and then stained with the "neutral red" staining procedure as described in the literature. The microtiter plates were evaluated by mean absorbance values of the wells containing the same concentration of the active substance. Photographs of the microtiter plates treated with the staining procedure are shown in Figures 2A and 2B, and the absorbance values measured at each well of the plates are given in Tables 1 and 2 below.

**Table 1**

| Conc. (µg/ml) | 250 | 125 | 60 | 30 | 15 | 7 | 3,5 | 1,75 | 0,8 | 0,4 | CV | CC |
|---|---|---|---|---|---|---|---|---|---|---|---|---|
| Nystatin+ CMC-1 | 0,1154 | 0,2035 | 0,3448 | 0,358 | 0,2889 | 0,2904 | 0,269 | 0,2602 | 0,2453 | 0,2415 | 0,2697 | 0,4774 |
| | 0,1093 | 0,1942 | 0,329 | 0,3666 | 0,3148 | 0,3238 | 0,2944 | 0,2753 | 0,2762 | 0,2369 | 0,2707 | 0,548 |
| | 0,1149 | 0,1788 | 0,464 | 0,351 | 0,3105 | 0,3305 | 0,355 | 0,3356 | 0,291 | 0,2576 | 0,2462 | 0,6307 |
| | 0,1169 | 0,1814 | 0,3886 | 0,3361 | 0,3018 | 0,3166 | 0,3425 | 0,3179 | 0,3113 | 0,2622 | 0,2739 | 0,5767 |
| Nystatin | 0,1248 | 0,4184 | 0,5119 | 0,5013 | 0,4352 | 0,4676 | 0,4578 | 0,4675 | 0,4576 | 0,49 | 0,2916 | 0,6327 |
| | 0,1 179 | 0,3538 | 0,4943 | 0,4855 | 0,4745 | 0,4816 | 0,4842 | 0,492 1 | 0,4735 | 0,5135 | 0,2874 | 0,5946 |
| | 0,1132 | 0,2153 | 0,4904 | 0,486 | 0,4673 | 0,4628 | 0,4935 | 0,6139 | 0,5606 | 0,5538 | 0,3104 | 0,6447 |
| | 0,1208 | 0,3089 | 0,4379 | 0,4654 | 0,4472 | 0,4521 | 0,4546 | 0,4641 | 0,525 | 0,5264 | 0,2829 | 0,5414 |

**Table 2**

| Conc. (µg/ml) | 250 | 125 | 60 | 30 | 15 | 7 | 3,5 | 1,75 | 0,8 | 0,4 | CV | CC |
|---|---|---|---|---|---|---|---|---|---|---|---|---|
| Nystatin+ VEVE | 0,1067 | 0,1684 | 0,4206 | 0,3216 | 0,3279 | 0,3128 | 0,3658 | 0,3731 | 0.3686 | 0,3770 | 0,3582 | 0,5311 |
| | 0,1317 | 0,1864 | 0,4201 | 0,3268 | 0,3460 | 0,3652 | 0,3611 | 0,3937 | 0,3925 | 0,3794 0.3794 | 0,3808 | 0,5261 |
| | 0,1157 | 0,1836 | 0,4258 | 0,3070 | 0,3172 | 0,3464 | 0,3993 | 0,4164 | 0,4070 | 0,3945 | 0,4130 | 0,6135 |
| | 0,1179 | 0,1663 | 0.5009 | 03493 | 0,3731 | 0.3438 | 0,3928 | 0,3958 | 0.3929 | 0,3726 | 0.3672 | 0,6017 |
| Nystatin | 0,1169 | 0,2694 | 0,5049 | 0,4755 | 0,4488 | 0,5339 | 0,4959 | 0,5183 | 0,5083 | 0,5464 | 0,6902 | 0,7504 |
| | 0,1160 | 0,3700 | 0,4794 | 0,4388 | 0,4530 | 0,4690 | 0,4595 | 0,4394 | 0,4830 | 0,4325 | 0,5967 | 0,6222 |
| | 0,1102 | 0,2478 | 0,4769 | 0,4528 | 0,4629 | 0,4709 | 0,4409 | 0,4745 | 0,4742 | 0,5204 | 0,5659 | 0,6712 |
| | 0,1212 | 0,3339 | 0,4536 | 0,4939 | 0,4069 | 0,4602 | 0,5149 | 0,4812 | 0,4718 | 0,5349 | 0,7398 | 0,6871 |

In Tables 1 and 2 above, column 11 contains virus controls (CV: cell cultures containing TCID₁₀₀ of untreated virus) and column 12 contains cell controls (CC: cell cultures without virus and drug). Where the corrected absorbance was greater than 0.05 positive, live cells (with intact morphology) were observable by microscopy in the culture at the end of the study (see Figure 4C). Said corrected absorbance was obtained by subtracting the mean value of the virus controls (CV) from the mean values of the samples containing the virus treated with the active substance, which CV mean value was calculated by ignoring the absorbance values of rows 5-8 of the CV column in Table 2, which are obviously the result of experimental error, since no virus was added to the cell cultures in these columns by mistake. TCID₁₀₀ of untreated virus used as a control caused complete cell death in tissue culture (Figure 4B).

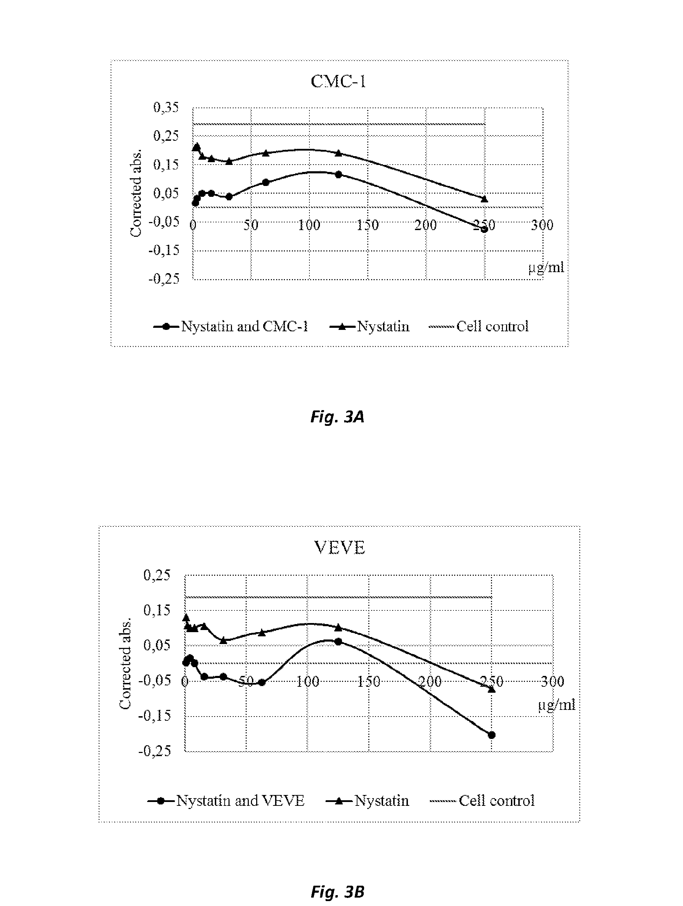

From the data in the tables, and from the graphs in Figures 3A and 3B, it can be seen that nystatin itself is toxic to the cultured cells (see curve 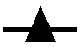 ), but there are concentrations where nystatin treatment reduces the infectious virus levels to such an extent (see curve 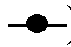 ) that viable cells also remain in the culture even at the end of the culture (incubation) period. Based on the study performed, the IC50 of nystatin is 60 µg/ml for the Wuhan strain and 125 µg/ml for the British mutant, which are lower than the usual concentration of nystatin in the intestinal tract (usually greater than 300 µg/ml), i.e. nystatin administered in the conventional way is expected to be effective in inhibiting the infectivity of the virus in the intestinal tract.

### REFERENCES

Abu-Farha, M.; Thanaraj, T.A.; Qaddoumi, M.G.; Hashem, A.; Abubaker, J.; Al-Mulla, F. (2020). The Role of Lipid Metabolism in COVID-19 Virus Infection and as a Drug Target. Int. J. Mol. Sci. 2020, 21, 3544. https://doi.org/10.3390/1ims21103544
Anand S, Mande SS. (2018). Diet, Microbiota and Gut-Lung Connection. Front Microbiol 2018; 9: 2147 (PMID: 30283410 DOI: 10.3389/fmicb.2018.02147)
Bhatt K, Agolli A, Patel MH, Garimella R, Devi M, Garcia E, Amin H, Domingue C, Guerra Del Castillo R, Sanchez-Gonzalez M. High mortality co-infections of COVID-19 patients: mucormycosis and other fungal infections. Discoveries (Craiova). 2021 Mar 31;9(1):e126. doi: 10.15190/d.2021.5. PMID: 34036149; PMCID: PMC8137279.
Carmen Racles, Mihai Mares, Liviu Sacarescu, A polysiloxane surfactant dissolves a poorly soluble drug (nystatin) in water Colloids and Surfaces A: Physicochemical and Engineering Aspects, Volume 443, 2014, Pages 233-239, ISSN 0927-7757, https://doi.org/10.1016/j.colsurfa.2013.11.010.
Corchuelo J, Ulloa FC. Oral manifestations in a patient with a history of asymptomatic COVID-19: Case report. Int J Infect Dis. 2020;100:154-157. doi:10.1016/j.ijid.2020.08.071
Coryell et al. (2021). A method for detection of SARS-CoV-2 RNA in healthy human stool: a validation study. Lancet Microbe 2021Published OnlineMarch 31, 2021 https://doi.org/10.1016/ S2666-5247(21)00059-8
Day TP, Sil D, Shukla NM, Anbanandam A, Day VW, David SA. Imbuing aqueous solubility to amphotericin B and nystatin with a vitamin. Mol Pharm. 2011 Feb 7;8(1):297-301. doi: 10.1021/mp100363f. Epub 2010 Dec 17. PMID: 21141891; PMCID: PMC3034802.
Dong M, Zhang J, Ma X, Tan J, Chen L, Liu S, Xin Y, Zhuang L. (2020). ACE2, TMPRSS2 distribution and extrapulmonary organ injury in patients with COVID-19. Biomed Pharmacother 2020; 131: 110678 (PMID: 32861070 DOI: 10.1016/j.biopha.2020.110678)
Geva-Zatorsky et al. (2017). Mining the human gut microbiota for immunomodulatory organisms. Cell 168, 928-943. 2017
Ghimire S, Sharma S, Patel A, Budhathoki R, Chakinala R, Khan H, Lincoln M, Georgeston M. Diarrhea Is Associated with Increased Severity of Disease in COVID-19: Systemic Review and Metaanalysis. SN Compr Clin Med. 2021 Jan 6:1-8;
Güemes-Villahoz, N., Burgos-Blasco, B., García-Feijoó, J. et al. (2020). Conjunctivitis in COVID-19 patients: frequency and clinical presentation. Graefes Arch Clin Exp Ophthalmol 258, 2501-2507.
Gupta S, Parker J, Smits S, Underwood J, Dolwani S. (2020). Persistent viral shedding of SARS-CoV-2 in faeces - a rapid review. Colorectal Dis 2020; 22: 611-620 (PMID: 32418307 DOI: 10.1111/codi. 15138)
Guyader et al. Role for Human Immunodeficiency Virus Type 1 Membrane Cholesterol in Viral Internalization. JOURNAL OFVIROLOGY, Oct. 2002, p. 10356-10364
Han et al. (2020). Digestive symptoms in COVID-19 patients with mild disease severity: clinical presentation, stool viral RNA testing, and outcomes. Am J Gastroenterol.; 115(6):916e923.
Jiang S, Hillyer C, Du L. (2020). Neutralizing antibodies against SARSCoV-2 and other human coronaviruses. Trends Immunol. 2020; 41(5):355e359. https://doi.org/10.1016/1.1t.2020.03.007.
Jin X, Lian et al. (2020). Epidemiological, clinical and virological characteristics of 74 cases of coronavirus-infected disease 2019 (COVID-19) with gastrointestinal symptoms. Gut. 2020;69(6):1002e1009.)
Kim et al. The architecture of SARS-CoV-2 transcriptome. Cell 2020;181:914-21.
Manenti A, Maggetti M, Casa E, Martinuzzi D, Torelli A, Trombetta CM, Marchi S, Montomoli E. Evaluation of SARS-CoV-2 neutralizing antibodies using a CPE-based colorimetric live virus micro-neutralization assay in human serum samples. J Med Virol. 2020 Oct;92(10):2096-2104. doi: 10.1002/jmv.25986. Epub 2020 May 17. PMID: 32383254; PMCID: PMC7267461.
Más, V & Melero, J.A. (2013). Entry of enveloped viruses into host cells: membrane fusion. Subcell Biochem. 68, 467-87. doi: 10.1007/978-94-007-6552-8_16. PMID: 23737062; PMCID: PMC7121288.
Mireille Guyader, Etsuko Kiyokawa, Laurence Abrami, Priscilla Turelli, Didier Trono (2002). Role for Human Immunodeficiency Virus Type 1 Membrane Cholesterol in Viral Internalization. Journal of Virology Oct 2002, 76 (20) 10356-10364; DOI: 10.1128/JVI.76.20.10356-10364.2002
Papoutsis et al. (2021). Detection of SARS-CoV-2 from patient fecal samples by whole genome sequencing. Gut Pathog (2021) 13:7
Pavlović N, Goločorbin-Kon S, Danić M, et al. Bile Acids and Their Derivatives as Potential Modifiers of Drug Release and Pharmacokinetic Profiles. Front Pharmacol. 2018;9:1283. Published 2018 Nov 8. doi:10.3389/fphar.2018.01283] [Mirza MA, Ahmad N, Agarwal SP, Mahmood D, Khalid Anwer M, Iqbal Z. Comparative evaluation of humic substances in oral drug delivery. Results Pharma Sci. 2011;1(1):16-26. Published 2011 Jul 12. doi:10.1016/j.rinphs.2011.06.001
Prakash S, Shukla S, Mishra H, Prakash O, Khan DN, Pandey A, Reddy DH, Jain A. SARS-CoV-2 - RNA persists longer in faecal sample as compared to nasal and throat swab samples of COVID-19 patients'; an observational study. Indian J Med Microbiol. 2021 Jan;39(1):122-124.
Razonable RR, Henault M, Watson HL, Paya CV. (2005). Nystatin induces secretion of interleukin (IL)-1beta, IL-8, and tumor necrosis factor alpha by a toll-like receptor-dependent mechanism. Antimicrob Agents Chemother. 2005 Aug;49(8):3546-9. doi: 10.1128/AAC.49.8.3546-3549.2005. PMID: 16048981; PMCID: PMC1196261.
Semis R, Kagan S, Berdicevsky I, Polacheck I, Segal E. Mechanism of activity and toxicity of Nystatin-Intralipid. Med Mycol. 2013 May;51(4):422-31. doi: 10.3109/13693786.2012.731712. Epub 2012 Oct 23. PMID: 23088298.
Silva et al. Competitive Binding of Cholesterol and Ergosterol to the Polyene Antibiotic Nystatin. A Fluorescence Study. Biophysical Journal Volume 90 May 2006 3625-3631
Szymczak et al. (2020). Utility of Stool PCR for the Diagnosis of COVID-19: Comparison of Two Commercial Platforms. Journal of Clinical Microbiology. September 2020 Volume 58 Issue 9 e01369-20
Wang, H., Yang, P., Liu, K. et al. (2008). SARS coronavirus entry into host cells through a novel clathrin- and caveolae-independent endocytic pathway. Cell Res 18, 290-301. https://doi.org/10.1038/cr.2008.15
Wang et al. (2020). Clinical Characteristics of 138 Hospitalized Patients With 2019 Novel Coronavirus-Infected Pneumonia inWuhan, China. JAMA. 2020;323(11):1061-1069. doi:10.1001/jama.2020.1585
Wang W, Xu Y, Gao R, Lu R, Han K, Wu G, Tan W. Detection of SARS-CoV-2 in Different Types of Clinical Specimens. JAMA. 2020 May 12;323(18):1843-1844;
Xiao F, Sun J, Xu Y, et al. Infectious SARS-CoV-2 in feces of patient with severe COVID-19. Emerg Infect Dis 2020;26:1920-1922
Zhang, X., Li, T., Chen, X. et al. (2018). Nystatin enhances the immune response against Candida albicans and protects the ultrastructure of the vaginal epithelium in a rat model of vulvovaginal candidiasis. BMC Microbiol 18, 166.
Zhang et al. (2020). Gastrointestinal symptoms, pathophysiology, and treatment in COVID-19. Genes Dis. 2020 Sep 5. doi: 10.1016/j.gendis.2020.08.013
Zuo T, Zhang F, Lui GCY, Yeoh YK, Li AYL, Zhan H, Wan Y, Chung ACK, Cheung CP, Chen N, Lai CKC, Chen Z, Tso EYK, Fung KSC, Chan V, Ling L, Joynt G, Hui DSC, Chan FKL, Chan PKS, Ng SC. (2020). Alterations in Gut Microbiota of Patients With COVID-19 During Time of Hospitalization. Gastroenterology 2020; 159: 944-955. e8 [PMID: 32442562 DOI: 10.1053/j.gastro.2020.05.048]

## Claims

1. Nystatin for use in the treatment of an infection caused by severe acute respiratory syndrome coronavirus 2 (SARS-CoV-2) in a human subject.

2. Nystatin for use according to claim 1, wherein the treatment is carried out by oral and/or intranasal administration, preferably such that nystatin comes in contact with the entire digestive tract and/or respiratory tract, including the oral cavity, pharynx, oesophagus, nasal cavity and lungs.

3. Nystatin for use according to claim 1, wherein the treatment is carried out by using rectal suppository.

4. Nystatin for use according to any of claims 1-3, wherein nystatin is formulated with a cosolvent, preferably in combination with pyridoxal phosphate, polysiloxane, bile acid, modified bile acid, fulvic acid, humic acid and/or intralipid.

5. Nystatin for use according to any of claims 1-4, wherein the subject is SARS-CoV-2 positive, preferably before the onset of symptoms or in the early stages of the disease, preferably wherein a replicable virus is detected in the digestive system of the subject.

6. Nystatin for use according to any of claims 1-2 and 4-5, wherein nystatin is formulated for oral and/or intranasal administration, preferably in oral pharmaceutical form selected from the group consisting of tablets, preferably film coated tablets, capsules, gels, suspensions, solutions, powders and inhalable dosage forms.

7. Nystatin for use according to any of claims 1-6, for administration for at least 5 days and/or up to 120 days, preferably for 90 days, preferably for 5-60 days, very preferably for 7-30 days or 10-21 days.

8. Nystatin for use according to any of claims 1-7, wherein nystatin is administered in a dose of at least 100,000, up to 1,000,000 international units (IU), preferably at least 200,000, up to 800,000 IU, very preferably at least 400,000, up to 600,000 IU, in particular about 500,000 IU.

9. Nystatin for use according to claim 8, wherein nystatin is administered to the human subject according to the following therapeutic regimen: administering a dose of 400,000, up to 600,000 IU, in particular about 500,000 IU, for 5-60 days, very preferably for 7-30 days, more preferably for 10-21 days.

10. Nystatin for use according to any of claims 1-9, wherein SARS-CoV-2 virus is detectable in the faecal sample of the subject at the beginning of the treatment.

11. Nystatin for use according to claim 10, wherein replicable SARS-CoV-2 virus is detectable in the faecal sample of the subject.

## Patentansprüche

1. Nystatin zur Verwendung bei der Behandlung einer Infektion, die durch das schwere akute respiratorische Syndrom Coronavirus 2 (SARS-CoV-2) bei einem Menschen verursacht wird.

2. Nystatin zur Verwendung nach Anspruch 1, wobei die Behandlung durch orale und/oder intranasale Verabreichung erfolgt, vorzugsweise so, dass Nystatin mit dem gesamten Verdauungstrakt und/oder den Atemwegen, einschließlich Mundhöhle, Rachen, Speiseröhre, Nasenhöhle und Lunge, in Kontakt kommt.

3. Nystatin zur Verwendung nach Anspruch 1, wobei die Behandlung unter Verwendung eines rektalen Zäpfchens durchgeführt wird.

4. Nystatin zur Verwendung nach einem der Ansprüche 1 bis 3, wobei Nystatin mit einem Co-Lösungsmittel formuliert ist, vorzugsweise in Kombination mit Pyridoxalphosphat, Polysiloxan, Gallensäure, modifizierter Gallensäure, Fulvosäure, Huminsäure und/oder Intralipid.

5. Nystatin zur Verwendung nach einem der Ansprüche 1 bis 4, wobei der Mensch SARS-CoV-2-positiv ist, vorzugsweise vor dem Auftreten von Symptomen oder im Frühstadium der Erkrankung, vorzugsweise wobei ein replikationsfähiges Virus im Verdauungssystem des Menschen nachgewiesen wird.

6. Nystatin zur Verwendung nach einem der Ansprüche 1-2 und 4-5, wobei Nystatin zur oralen und/oder intranasalen Verabreichung formuliert ist, vorzugsweise in einer oralen pharmazeutischen Form, ausgewählt aus der Gruppe bestehend aus Tabletten, vorzugsweise Filmtabletten, Kapseln, Gelen, Suspensionen, Lösungen, Pulvern und inhalierbaren Darreichungsformen.

7. Nystatin zur Verwendung nach einem der Ansprüche 1-6, zur Verabreichung über mindestens 5 Tage und/oder bis zu 120 Tage, vorzugsweise über 90 Tage, vorzugsweise über 5 bis 60 Tage, sehr bevorzugt über 7 bis 30 Tage oder 10 bis 21 Tage.

8. Nystatin zur Verwendung nach einem der Ansprüche 1-7, wobei Nystatin in einer Dosis von mindestens 100.000 bis zu 1.000.000 internationalen Einheiten (IE), vorzugsweise mindestens 200.000 bis zu 800.000 IE, sehr bevorzugt mindestens 400.000 bis zu 600.000 IE, insbesondere etwa 500.000 IE, verabreicht wird.

9. Nystatin zur Verwendung nach Anspruch 8, wobei Nystatin dem Menschen nach folgenden therapeutischen Schema verabreicht wird: Verabreichung einer Dosis von 400.000 bis zu 600.000 IE, insbesondere etwa 500.000 IE, über einen Zeitraum von 5 bis 60 Tagen, sehr bevorzugt über einen Zeitraum von 7 bis 30 Tagen, noch bevorzugter über einen Zeitraum von 10 bis 21 Tagen.

10. Nystatin zur Verwendung nach einem der Ansprüche 1-9, wobei das SARS-CoV-2-Virus in der Stuhlprobe des Menschen zu Beginn der Behandlung nachweisbar ist.

11. Nystatin zur Verwendung nach Anspruch 10, wobei replizierbares SARS-CoV-2-Virus in der Stuhlprobe des Menschen nachweisbar ist.

## Revendications

1. Nystatine pour une utilisation dans le traitement d'une infection causée par le coronavirus 2 du syndrome respiratoire aigu sévère (SARS-CoV-2) chez un sujet humain.

2. Nystatine pour une utilisation selon la revendication 1, dans laquelle le traitement est effectué par administration orale et/ou intranasale, de préférence de manière à ce que la nystatine entre en contact avec l'ensemble du tube digestif et/ou des voies respiratoires, y compris la cavité buccale, le pharynx, l'œsophage, la cavité nasale et les poumons.

3. Nystatine pour une utilisation selon la revendication 1, dans laquelle le traitement est effectué à l'aide d'un suppositoire rectal.

4. Nystatine pour une utilisation selon l'une quelconque des revendications 1 à 3, dans laquelle la nystatine est formulée avec un cosolvant, de préférence en association avec du phosphate de pyridoxal, du polysiloxane, de l'acide biliaire, de l'acide biliaire modifié, de l'acide fulvique, de l'acide humique et/ou de l'intralipide.

5. Nystatine pour une utilisation selon l'une quelconque des revendications 1 à 4, dans laquelle le sujet est positif au SARS-CoV-2, de préférence avant l'apparition des symptômes ou aux premiers stades de la maladie, et de préférence lorsqu'un virus réplicable est détecté dans le système digestif du sujet.

6. Nystatine pour une utilisation selon l'une quelconque des revendications 1 à 2 et 4 à 5, dans laquelle la nystatine est formulée pour administration orale et/ou intranasale, de préférence sous forme pharmaceutique orale choisie dans le groupe constitué par des comprimés, de préférence des comprimés pelliculés, des gélules, des gels, des suspensions, des solutions, des poudres et des formes posologiques inhalables.

7. Nystatine pour une utilisation selon l'une quelconque des revendications 1 à 6, pour une administration pendant au moins 5 jours et/ou jusqu'à 120 jours, de préférence pendant 90 jours, de préférence pendant 5 à 60 jours, très préférentiellement pendant 7 à 30 jours ou 10 à 21 jours.

8. Nystatine pour une utilisation selon l'une quelconque des revendications 1 à 7, dans laquelle la nystatine est administrée à une dose d'au moins 100 000, jusqu'à 1 000 000 d'unités internationales (UI), de préférence d'au moins 200 000, jusqu'à 800 000 UI, très préférentiellement d'au moins 400 000, jusqu'à 600 000 UI, en particulier d'environ 500 000 UI.

9. Nystatine pour une utilisation selon la revendication 8, dans laquelle la nystatine est administrée au sujet humain selon le schéma thérapeutique suivant : administration d'une dose de 400 000, jusqu'à 600 000 UI, en particulier d'environ 500 000 UI, pendant 5 à 60 jours, très préférentiellement pendant 7 à 30 jours, plus préférentiellement pendant 10 à 21 jours.

10. Nystatine pour une utilisation selon l'une quelconque des revendications 1 à 9, dans laquelle le virus SARS-CoV-2 est détectable dans l'échantillon de selles du sujet au début du traitement.

11. Nystatine pour une utilisation selon la revendication 10, dans laquelle un virus SARS-CoV-2 réplicable est détectable dans l'échantillon de selles du sujet.
